# EUROPEAN PATENT SPECIFICATION

(11) **EP 1 013 648 B1**
(45) Date of publication and mention of the grant of the patent: **19.03.2003**
(21) Application number: 99125436.8
(22) Date of filing: 20.12.1999
(51) Int. Cl.: C07D 305/06

(54) **Method for storage stabilization of compounds with oxetane rings**
Verfahren zur Lagerstabilisierung von Verbindungen mit Oxetan Ringen
Méthode de stabilisation pour le stockage de composés à cycle oxétane

(30) Priority: 21.12.1998 JP 36286198
(43) Date of publication of application: 28.06.2000
(73) Proprietor: Toagosei Co., Ltd., Minato-ku, Tokyo 105-8419 (JP)
(72) Inventor: Ito, Naokazu, Minato-ku, Nagoya-shi, Aichi, 455-0027 (JP); Sasaki, Hiroshi, Minato-ku, Nagoya-shi, Aichi, 455-0027 (JP); Kuriyama, Akira, Minato-ku, Nagoya-shi, Aichi, 455-0027 (JP)
(74) Representative: WILHELMS, KILIAN & PARTNER Patentanwälte

(56) References cited:
- PATENT ABSTRACTS OF JAPAN vol. 1998, no. 05, 30 April 1998 (1998-04-30) & JP 10 007669 A (TOAGOSEI CO LTD), 13 January 1998 (1998-01-13)

## Description

The present invention relates to a method for storage stabilization of compounds with oxetane rings (hereunder referred to as "oxetane compounds") that are useful as ring opening polymerizable monomers.

Oxetane compounds are 4-membered cyclic ethers that are known to undergo ring opening polymerization with cationic polymerization catalysts such as Lewis acids. Oxetane compounds are described, for example, in ENCYCLOPEDIA OF POLYMER SCIENCE AND ENGINEERING, Vol.10, p.653-670. These oxetane compounds are used as raw materials, intermediates and additives for resins, coating agents, adhesives and the like, by taking advantage of their ring opening polymerization properties.

In Patent Abstracts of Japan vol. 1998, no. 05, 1998 & JP-A-10 007669 a process of synthesis of oxetane compounds by reaction of a triol with an alkyl or alkylene carbonate followed by decarboxylation in presence of a basic catalyst is disclosed, whereas no indication concerning problems of storage stabilization of such compounds can be found.

However, when oxetane compounds are stored over long periods, ring opening polymerization occurs as a result of contamination by trace impurities during production, causing a problem in terms of product quality because of clouding due the resulting polymers.

The method for production of oxetane compounds described by Pattison in the Journal of the American Chemical Society (J. Am. Chem. Soc.), 79, 3455 (1957) is a method of synthesizing oxetane compounds by ester exchange reaction between 1,3-diols and dialkylcarbonates to obtain 6-membered carbonates, and then heating and decarboxylating the product. Research by the present inventors, however, has demonstrated that oxetane compounds produced by this process, such as 3-methyl-3-hydroxymethyloxetane and 3-ethyl-3-hydroxymethyloxetane, often undergo clouding during long-term storage.

It is an object of the present invention to provide a method for storage stabilization of oxetane compounds that prevents clouding of oxetane compounds and avoids their quality deterioration over long periods.

As a result of diligent research aimed at solving the aforementioned problems, the present inventors have completed the present invention upon finding that addition of basic compounds can improve the storage stability of oxetane compounds without impairing their functions including ring opening polymerization.

In other words, the present invention relates to a method according to claim 1, further preferred embodiments being disclosed in claims 2 to 5.

The present invention provides means for solving the problems described above, and is briefly described as claims. The nature, principle and utility of the invention will become more apparent from the following detailed description with reference to the accompanying drawings.

The invention will now be explained in greater detail.

As oxetane compounds of the invention there may be mentioned 3,3-dialkyloxetanes, 3-alkyl-3-hydroxymethyloxetanes, 3-alkyl-3-chloromethyloxetanes, 3-alkyl-3-alkyloxyoxetanes, 3-alkyl-3-phenyloxyoxetanes, bis(3-alkyloxetan-3-ylmethyl) ethers, 2-alkyloxetanes, 2-phenyloxetanes, 2,2'-dialkyloxetanes, 2,3-dialkyloxetanes and 2-phenyl-3,3-dialkyloxetanes, among which are preferred oxetane compounds with substituents at the 3- or 2-position thereof as they more readily exhibit the effect of the invention, and 3-alkyl-3-hydroxymethyloxetanes are especially preferred.

As the basic compounds for use in the invention there may be used basic inorganic compounds such as alkali metal hydroxides and the like, and basic organic compounds such as amine compounds and the like. As basic inorganic compounds there may be mentioned lithium hydroxide, sodium hydroxide, potassium hydroxide, sodium carbonate, sodium bicarbonate, magnesium hydroxide, calcium hydroxide and potassium carbonate, among which alkali metal hydroxides such as lithium hydroxide, sodium hydroxide and potassium hydroxide are preferred from the standpoint of solubility in oxetane compounds.

As basic organic compounds there may be mentioned amines as well as nitrogenous heterocyclic compounds such as quinolines and quinolidines, among which amine compounds are preferred from the standpoint of compatibility with oxetane compounds; as examples there may be mentioned octylamine, naphthylamine, xylenediamine, dibenzylamine, diphenylamine, dibutylamine, dioctylamine, dimethylaniline, quinacridine, tributylamine, trioctylamine, tetramethylethylenediamine, tetramethyl-1,6-hexamethylenediamine, hexamethylenetetraamine and triethanolamine.

Oxetane compounds are often used in resins and coating agents, taking advantage of their property whereby ring opening polymerization is caused by cationic polymerization catalysts such as Lewis acids, in which case the basic compound functions as an inhibitor on ring opening polymerization. The amount of the basic compound to be added in order to ensure stable storage without impairing the function of the oxetane compound is preferably in a range of 0.1 ppm by weight to 1000 ppm by weight as the amount of added basic compound with respect to the oxetane compound, and more preferably in a range of 1 ppm by weight to 1000 ppm by weight. If the amount added is less than 0.1 ppm by weight it is not possible to achieve adequate storage stability, and even if it exceeds 1000 ppm by weight there is no further improvement in storage stability, while the ring opening polymerization may be inhibited instead, possibly impairing the function of the oxetane compound.

Methods of addition of the basic compound to the oxetane compound are not particularly limited, and it may be accomplished with normal mixing conditions. The basic compound may be used in dilution with water or the like, but it is preferably added directly.

### Examples

The present invention will now be explained in further detail by way of examples and comparative examples.

### Example 1

Potassium hydroxide was added as a storage stabilizer at 1 ppm by weight, 10 ppm by weight and 100 ppm by weight to 3-ethyl-3-hydroxymethyloxetane produced by the above-mentioned Pattison method using trimethylolpropane and diethyl carbonate as the raw materials, and each mixture was stored in a thermostatic chamber at 25°C.
1) The storage stability was evaluated by the following method, and the results are shown in Table 1.
   The mixture was diluted 100-fold with distilled water and the transmittance was measured at a wavelength of 600 nm with a spectrophotometer. The same test was conducted again after 40 days and after 60 days, after which the storage stability was evaluated based on the changes in the values.
2) The polymerization was evaluated by the following method, and the results are shown in Table 1.

An alicyclic epoxy UVR-6110 (trade name) by Union Carbide Co. and a photoinitiator UVI-6990 (trade name) by Union Carbide Co. were mixed with the 3-ethyl-3-hydroxymethyloxetane obtained above at a weight ratio of 3-ethyl-3-hydroxymethyloxetane/UVR-6110/UVI-6990 = 20/80/3. The resulting composition was coated onto a steel sheet to a thickness of about 20 microns. Using a conveyer-type ultraviolet irradiation apparatus equipped with an 80 w/cm condensing high-pressure mercury lamp (lamp height = 10 cm, irradiation intensity: 310 mW/cm², 76 mJ/cm²), the conveyer speed was changed and measurement was made of the maximum conveyer speed (m/min) at which stickiness was eliminated from the surface thereof. The condition of any stickiness on the cured surface obtained at that time was also evaluated.

A faster conveyer speed indicates earlier curability.

### Comparative Example 1

A test was conducted in the same way as Example 1 except that no potassium hydroxide was added; the results are shown in Table 1.

### Comparative Example 2

A test was conducted in the same way as Example 1 except that potassium hydroxide was added at 10,000 ppm by weight; the results are shown in Table 1.

### Example 2

A test was conducted in the same way as Example 1 except that tri-n-butylamine was added at 10 ppm by weight and 100 ppm by weight; the results are shown in Table 1.

**Table 1**

| | Additive | Amount added (ppm) | Storage stability test | | | Polymerization | |
|---|---|---|---|---|---|---|---|
| | | | Change in transmittance with time (%) | | | Conveyer speed (m/min) | Stickiness on surface |
| | | | Day 0 | Day 40 | Day 60 | | |
| Example 1 | KOH | 1 | 100 | 100 | 99.1 | 30 | no |
| | " | 10 | 100 | 100 | 100 | 30 | no |
| | " | 100 | 100 | 100 | 100 | 30 | no |
| Example 2 | TBuA | 10 | 100 | 100 | 100 | 30 | no |
| | " | 100 | 100 | 100 | 100 | 30 | no |
| Comp. Ex. 1 | none | 0 | 100 | 97.6 | 92.9 | 30 | no |
| Comp. Ex. 2 | KOH | 10,000 | 100 | 100 | 100 | 10 | yes |

| | | | | | | | |
|---|---|---|---|---|---|---|---|
| KOH: potassium hydroxide, | | | | | | | |
| TBuA: tri-n-butylamine | | | | | | | |
| Polymerization: conveyer speed (m/min) and condition of surface stickiness | | | | | | | |

### Example 3

Sodium hydroxide was added as a storage stabilizer at 10 ppm by weight and 100 ppm by weight to 3-methyl-3-hydroxymethyloxetane produced by the Pattison method using trimethylolethane and diethyl carbonate as the raw materials, and each mixture solution was stored in a thermostatic chamber at 25°C. The stability and polymerization were evaluated in the same way as Example 1, and the results are shown in Table 2. However, the changes in transmittance with time were judged after 20 days arid 40 days.

### Comparative Example 3

A test was conducted in the same way as Example 3 except that no sodium hydroxide was added; the results are shown in Table 2.

### Comparative Example 4

A test was conducted in the same way as Example 3 except that sodium hydroxide was added at 10,000 ppm by weight; the results are shown in Table 2.

**Table 2**

| | Additive | Amount added (ppm) | Storage stability test | | | Polymerization | |
|---|---|---|---|---|---|---|---|
| | | | Change in transmittance with time (%) | | | Conveyer speed (m/min) | Stickiness on surface |
| | | | Day 0 | Day 20 | Day 40 | | |
| Example 3 | NaOH | 10 | 100 | 100 | 100 | 30 | no |
| | " | 100 | 100 | 100 | 100 | 30 | no |
| Comp. Ex. 3 | none | 0 | 100 | 92.5 | 87.3 | 30 | no |
| Comp. Ex. 4 | NaOH | 10,000 | 100 | 100 | 100 | 10 | yes |

| | | | | | | | |
|---|---|---|---|---|---|---|---|
| NaOH: sodium hydroxide | | | | | | | |
| Polymerization: conveyer speed (m/min) and condition of surface stickiness | | | | | | | |

According to the method of the invention, it is possible to improve the storage stability of oxetane compounds without impairing their ring opening polymerization, by addition of basic compounds to the oxetane compounds.

While there has been described what are at present considered to be preferred embodiments of the invention, it will be understood that various modifications may be made thereto, and it is intended that the appended claims cover all such modifications as fall within the true spirit and scope of the invention.

## Claims

1. A method for storage stabilization of a compound with an oxetane ring or oxetane rings, which is **characterized by** adding a basic compound to the compound with an oxetane ring or rings in an amount of 0.1 to 1,000 ppm by weight per the compound with an oxetane ring or oxetane rings.

2. A method according to claim 1, wherein the basic compound is at least one selected from the group consisting of an alkali metal hydroxide and an amine compound.

3. A method according to claim 1 or 2, wherein the compound with an oxetane ring of oxetane rings has a substituent at the 2- or 3-position thereof.

4. A method according to any one of claims 1 to 3, wherein the compound with an oxetane ring or rings is a 3-alkyl-3-hydroxymethyloxetane.

5. A method according to claim 1 to 4, wherein the basic compound is added in an amount of 1 to 1,000 ppm by weight per the compound with an oxetane ring or oxetane rings.

## Patentansprüche

1. Verfahren zur Lagerungsstabilisierung einer Verbindung mit einem Oxetanring oder Oxetanringen, **dadurch gekennzeichnet, dass** eine basische Verbindung zu der Verbindung mit einem Oxetanring oder Oxetanringen in einer Menge von 0,1 bis 1.000 ppm Gewichtsteilen, bezogen auf die Verbindung mit einem Oxetanring oder Oxetanringen, gegeben wird.

2. Verfahren gemäß Anspruch 1, wobei es sich bei der basischen Verbindung um ein Alkalimetallhydroxid und/oder eine Aminverbindung handelt.

3. Verfahren gemäß Anspruch 1 oder 2, wobei die Verbindung mit einem Oxetanring oder Oxetanringen einen Substituenten an ihrer 2- oder 3-Position aufweist.

4. Verfahren gemäß wenigstens einem der Ansprüche 1 bis 3, wobei die Verbindung mit einem Oxetanring oder Oxetanringen ein 3-Alkyl-3-hydroxymethyloxetan ist.

5. Verfahren gemäß wenigstens einem der Ansprüche 1 bis 4, wobei die basische Verbindung in einer Menge von 1 bis 1.000 ppm Gewichtsteilen, bezogen auf die Verbindung mit einem Oxetanring oder Oxetanringen, zugegeben wird.

## Revendications

1. Méthode de stabilisation pour le stockage d'un composé à cycle oxétane ou à cycles oxétanes, **caractérisée par** l'addition d'un composé basique au composé avec un cycle ou des cycles oxétanes, dans une quantité allant de 0,1 à 1,000 ppm en poids du composé avec un cycle oxétane ou avec des cycles oxétanes.

2. Méthode selon la revendication 1, dans laquelle le composé basique est au moins un composé sélectionné à partir du groupe constitué de l'hydroxyde de métal alcalin et d'un composé amine.

3. Méthode selon la revendications 1 ou 2, dans laquelle le composé avec un cycle oxétane ou des cycles oxétanes a un groupe de substitution à la position 2 ou 3.

4. Méthode selon l'une quelconque des revendications 1 à 3, dans laquelle le composé ayant un cycle ou des cycles oxétanes est un 3 - alkyle - 3 - hydroxyméthyloxétane.

5. Méthode selon l'une des revendications 1 à 4, dans laquelle le composé basique est ajouté dans une quantité allant de 1 à 1.000 ppm en poids du composé avec un cycle oxétane ou avec des cycles oxétanes.
